**Europäisches Patentamt**

**European Patent Office**

**Office européen des brevets**

(19)

(11) Numéro de publication: **0 316 992**
**A1**

# (12) DEMANDE DE BREVET EUROPEEN

(21) Numéro de dépôt: **88202491.2**

(22) Date de dépôt: **08.11.88**

(51) Int. Cl.4: **A61L 27/00 , B29C 55/30**

(30) Priorité: **19.11.87 FR 8716147**

(43) Date de publication de la demande:
**24.05.89 Bulletin 89/21**

(84) Etats contractants désignés:
**AT BE CH DE ES FR GB GR IT LI LU NL SE**

(71) Demandeur: **SOLVAY & Cie (Société Anonyme)**
**Rue du Prince Albert, 33**
**B-1050 Bruxelles(BE)**

(72) Inventeur: **Delcommune, Luc**
**Via Timavo, 59**
**I-34074 Monfalcone (GO)(IT)**
Inventeur: **Ghyselinck, Philippe**
**Avenue des Tarins, 17**
**B-1420 Braine-l'Alleud(BE)**

(54) **Article en polymère d'acide lactique utilisable notamment comme prothèse biodégradable et procédé pour sa réalisation.**

(57) Article en polymère d'acide lactique utilisable notamment comme prothèse biodégradable présentant à 23°C un module de rigidité en traction supérieur à 5000 MPa et une résilience en traction supérieure à 500 kJ/m² et un retrait thermique inférieur à 15 % à 130°C.

L'article est réalisé par forgeage d'une ébauche à une température comprise entre 80 et 170°C, avec un taux d'étirage de 200 à 1000 %.

EP 0 316 992 A1

EP 0 316 992 A1

## Article en polymère d'acide lactique utilisable notamment comme prothèse biodégradable et procédé pour sa réalisation

La présente invention concerne à titre principal un article en polymère d'acide lactique utilisable notamment comme prothèse biodégradable, en particulier en chirurgie osseuse, et présentant des propriétés mécaniques améliorées.

Une prothèse biodégradable doit répondre à deux exigences; d'une part, elle doit être suffisamment rigide pour que l'union entre les parties osseuses fracturées puisse avoir lieu au début de la guérison et d'autre part, elle doit être suffisamment souple pour stimuler la formation d'un cal osseux par l'intermédiaire des sollicitations exercées sur les membres.

Les polymères d'acide lactique et en particulier l'homopolymère d'acide L-lactique qui ont déjà été envisagés pour la réalisation de telles prothèses ont été jugés trop rigides et surtout trop cassants (Biomaterials, 1980, 3, p. 276). Pour remédier à ces déficiences mécaniques, il a alors été proposé de renforcer ce matériau par des fibres telles que les fibres de carbone (Orthoped. Rev. 1981, 10, p. 41), de poly-acide glycolique (Biomaterials 1980, p. 271) ou encore de métaphosphate de calcium (Demande de brevet européen 0146398). En opérant de cette façon, on arrive certes à produire des prothèses ayant une rigidité acceptable mais on constate que celles-ci restent fragiles. En outre, il est bien évident que la réalisation de telles prothèses est délicate et coûteuse.

La demanderesse a maintenant constaté qu'il est possible de réaliser directement à partir d'un polymère d'acide lactique des articles tels que des prothèses biodégradables présentant des caractéristiques mécaniques remarquables qui rendent ceux-ci particulièrement efficaces notamment en chirurgie osseuse.

La présente invention concerne dès lors un article en polymère d'acide lactique utilisable notamment comme prothèse biodégradable présentant à 23° C un module de rigidité en traction supérieur à 5000 MPa et, de préférence, supérieur à 7500 MPa et une résilience en traction supérieure à 500 kJ/m$^2$ et, de préférence, supérieure à 3000 kJ/m$^2$. Dans un mode d'exécution qui est préféré, l'article conforme à l'invention présente, en outre, un retrait thermique inférieur à 15 % à 130° C.

Les polymères d'acide lactique utilisés pour la réalisation des articles selon l'invention sont choisis parmi les polymères thermoplastiques résultant de l'homopolymérisation d'acide-L-lactique, de la copolymérisation d'acide-L-lactique et d'acide-D-lactique à raison de 99 à 50 % d'acide-L-lactique et/ou encore avec d'autres monomères copolymérisables. Ces autres monomères copolymérisables peuvent être, par exemple, la bêta-propiolactide, la tétraméthylglycolide, la bêta-butyrolactone, la gamma-butyrolactone, la pivalolactone, l'acide alpha-hydroxyacétique, l'acide alpha-hydroxybutyrique, l'acide alpha-hydroxyisobutyrique, l'acide alpha-hydroxyvalérique, l'acide alpha-hydroxyisovalérique, l'acide alpha-hydroxycaproïque, l'acide alpha-hydroxyisocaproïque, l'acide alpha-hydroxy-alpha-éthylbutyrique, l'acide alpha-hydroxyheptanoïque, l'acide alpha-hydroxystéarique, etc.

Les copolymères résultants peuvent être aussi bien des copolymères statistiques que des copolymères appelés "à blocs". Ces copolymères à blocs sont constitués par une succession de segments de chaînes de longueur variable, chaque segment consistant en un homopolymère d'un monomère ou en un copolymère statistique comprenant deux ou plusieurs comonomères tels que définis ci-dessus.

Les polymères biodégradables préférés pour la réalisation des prothèses selon l'invention sont les homopolymères d'acide-L-lactique et les copolymères d'acide-L-lactique et d'acide-D-lactique comprenant de 99 à 50 % d'acide-L-lactique. Le recours à un copolymère d'acide-L-lactique et d'acide-D-lactique présente l'avantage de permettre un réglage de la durée de biodégradation en agissant sur la teneur en unités dérivées de l'acide-D-lactique.

Pour des raisons de tenue des propriétés mécaniques dans le temps des prothèses, les polymères d'acide lactique convenant particulièrement bien pour l'invention sont ceux dont le poids moléculaire moyen est supérieur à 250.000 et, de préférence, à 400.000. Ce poids moléculaire moyen est défini par la relation $\overline{M}_w = \Sigma$ ni Mi$^2$/$\Sigma$ ni Mi dans laquelle ni représente le nombre de molécules de poids moléculaire Mi. Ce poids moléculaire moyen est déterminé de manière connue par les méthodes cryoscopiques et ébullioscopiques, par la méthode des groupes terminaux ou encore par osmométrie.

L'article conforme à la présente invention peut être notamment réalisé en extrudant une ébauche en polymère d'acide lactique à 170-250° C, en conditionnant thermiquement l'ébauche à une température de 80-170° C, puis en soumettant celle-ci à un étirage de 200-1000 %. Avantageusement, l'article peut être enfin recuit sous tension à une température d'au moins 130° C.

L'ébauche peut être réalisée par les techniques de mise en oeuvre classiques, généralement l'extrusion ou l'injection. Toutefois, il peut être fait appel à d'autres techniques comme le frittage par exemple.

L'ébauche peut avoir une section droite quelconque : circulaire, ovale, polygonale, etc. De préférence, l'ébauche se présente sous forme d'un cylindre ou d'une plaquette.

La température de conditionnement de l'ébauche avant étirage se situe 10 à 100° C en-dessous de la température de fusion de la matière, soit à une température de 80 à 170° C. Le conditionnement peut être réalisé dans une étuve ou dans le fourreau d'une extrudeuse dont on a extrait la vis. Le temps de conditionnement de l'ébauche doit être au moins égal au temps nécessaire à l'uniformisation de la température dans la masse de la matière. Les matières facilement oxydables à chaud doivent être protégées par un gaz neutre, comme l'azote par exemple. Le temps de conditionnement est généralement compris entre 5 et 120 minutes et, de préférence, entre 15 et 90 minutes.

L'ébauche ainsi conditionnée est ensuite soumise à un étirage axial comme décrit précédemment. Dans un mode de réalisation qui est préféré, le conditionnement thermique est effectué dans le fourreau d'une extrudeuse comme mentionné ci-avant et l'étape d'étirage est réalisée en tirant l'ébauche à travers une filière convergente montée sur l'extrudeuse, la section de sortie correspondant à celle souhaitée pour l'article à produire. Le rapport entre la section initiale de l'ébauche et la section de sortie de la filière convergente donne le rapport d'étirage appliqué à l'ébauche. De préférence, ce rapport est de 200 à 1000 %. La vitesse d'étirage est comprise entre 50 et 1000 mm/min et, de préférence, entre 100 et 500 mm/min.

Comme tous les procédés d'orientation moléculaire, l'étirage de l'ébauche selon le mode de réalisation ainsi décrit, a pour effet de provoquer l'apparition de tensions internes dans la matière constitutive. Lorsque la prothèse biodégradable ainsi réalisée est appelée à être ultérieurement stérilisée à une température voisine ou supérieure à la température d'étirage, il est préférable de soumettre l'article à une étape supplémentaire de stabilisation thermique car la température limite d'utilisation de l'article sans stabilisation préalable se situe environ 20 à 40° C en dessous de la température d'étirage. Suivant l'importance des articles, une stabilisation satisfaisante peut être obtenue par un recuit de 30 à 90 minutes à une température supérieure de 5 à 20° C à la température de stérilisation des prothèses biodégradables.

Dans certains cas, la stabilisation peut également être utilisée pour obtenir des cotes de tolérances serrées en effectuant un contrôle des dimensions pendant la stabilisation. Ainsi, par exemple, le diamètre extérieur d'un article cylindrique peut être fixé avec précision en enfilant un tube calibré autour de l'article pendant la stabilisation.

Bien que les articles suivant l'invention puissent être réalisés sous diverses formes, la forme cylindrique a été choisie dans les exemples qui vont suivre, cette forme convenant particulièrement pour des prothèses biodégradables devant être implantées à l'intérieur des parties osseuses fracturées.

Les exemples suivants servent à illustrer l'invention.

Exemple 1

On alimente une extrudeuse monovis de laboratoire équipée d'une filière à jonc de 17,2 mm de diamètre au moyen d'un homopolymère d'acide-L-lactique de poids moléculaire moyen de 500.000 et dont la température de fusion s'élève à 172° C, les températures appliquées dans les différentes zones de l'extrudeuse étant les suivantes : .
- 165° C dans la zone d'alimentation
- 180° C dans la zone de compression
- 180° C dans la zone de dosage
- 180° C pour la filière de sortie.

Le jonc cylindrique liquide sortant de l'extrudeuse après refroidissement et solidification présente un diamètre égal à 16,5 mm.

Le jonc est ensuite conditionné thermiquement dans le fourreau d'une extrudeuse dépourvue de vis et munie d'une filière ronde convergente de 6 mm de diamètre de sortie. La température de conditionnement du jonc est fixée à 150° C et la durée de conditionnement à 60 minutes. Ensuite, le jonc conditionné est tiré à travers la filière convergente de sortie avec une vitesse d'étirage de 200 mm/min. Le diamètre du jonc étiré ainsi produit est égal à 5,5 mm, ce qui correspond à un rapport d'étirage de 9. Le jonc étiré est ensuite recuit pendant 60 minutes à 130° C sous tension.

Les propriétés mécaniques de l'article ainsi obtenu mesurées suivant la norme ISO 527 donnent les résultats suivants :

| - module d'élasticité en traction | = 8830 MPa |
|---|---|
| - tension de rupture | = 176 MPa |
| - allongement à la rupture | = 11 % |
| - tension maximale élastique | = 182 MPa |
| - allongement à la tension maximale élastique | = 8% |

La résilience en traction mesurée suivant la norme DIN 53448 s'élève à 3600 kJ/m².

Exemple 1R

Cet exemple est donné à titre de comparaison.

Le jonc cylindrique de 16,5 mm de diamètre obtenu à l'exemple 1 est pressé à 195° C pour former une plaque de 2 mm d'épaisseur, puis refroidi et solidifié.

Les propriétés mécaniques mesurées suivant la norme ISO 527 donnent les résultats suivants :

| - module d'élasticité en traction | = 3670 MPa |
|---|---|
| - tension maximale élastique | = 66 MPa |
| - allongement à la tension maximale élastique | = 2,4 % |
| - tension de rupture | = 59 MPa |
| - allongement à la rupture | = 3,6 %. |

La résilience en traction mesurée suivant la norme DIN 53448 est égale à 94 kJ/m². La plaque ainsi obtenue est donc caractérisée par une grande fragilité qui la rend inapte pour une utilisation comme prothèse en chirurgie osseuse.

**Revendications**

1 - Article en polymère d'acide lactique utilisable notamment comme prothèse biodégradable présentant à 23° C un module de rigidité en traction supérieur à 5000 MPa et une résilience en traction supérieure à 500 kJ/m².

2 - Article suivant la revendication 1 caractérisé en ce qu'il présente en outre un retrait thermique inférieur à 15 % à 130° C.

3 - Article suivant la revendication 1 caractérisé en ce que le polymère d'acide lactique est un homopolymère d'acide-L-lactique.

4 - Article suivant la revendication 1 caractérisé en ce que le polymère d'acide lactique est un copolymère d'acide-L-lactique et d'acide-D-lactique comprenant de 99 à 50 % d'unités dérivées de l'acide-L-lactique.

5 - Article suivant la revendication 1 caractérisé en ce que le polymère d'acide lactique est un copolymère d'acide-L-lactique avec au moins un autre monomère copolymérisable.

6 - Procédé pour réaliser un article selon les revendications 1 à 5 caractérisé en ce que le polymère d'acide lactique est extrudé à 170-250° C, pour former une ébauche qui est ensuite soumise à un conditionnement thermique à une température de 80-170° C, puis à un étirage de 200-1000 %.

7 - Procédé pour réaliser un article selon la revendication 6 caractérisé en ce que l'article est ensuite soumis à un recuit sous tension à une température d'au moins 130° C.

8 - Procédé pour réaliser un article selon la revendication 6 caractérisé en ce que l'étirage est réalisé par tirage de l'ébauche thermiquement conditionnée au travers d'une filière convergente dont la section de sortie correspond à celle souhaitée pour l'article à produire.

4

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | FR-A-2 088 548 (ETHICON)<br>* Revendications 1,11,13; page 1, ligne 7; page 3, lignes 29-40; page 4, lignes 1-9; exemples V-XIII; page 9, lignes 23-27 * | 1,4,5 | A 61 L 27/00<br>B 29 C 55/30 |
| Y | --- | 6-8 | |
| X | GB-A-1 123 445 (DU PONT)<br>* Revendications 1,2,3,5,15,16; exemples I-IX; page 10, lignes 13-17 * | 1,2,4,5 | |
| Y | --- | 6-8 | |
| X | FR-A-1 478 695 (ETHICON)<br>* Revendications 1,3; exemple 1 * | 1,3,5 | |
| Y | --- | 6-8 | |
| Y | EP-A-0 133 355 (NATIONAL RESEARCH)<br>* Revendications 1,13; page 3, lignes 11,12,26,27 *<br>--- | 6-8 | |
| A | FR-A-2 156 513 (AMERICAN CYANAMID)<br>* Page 10, ligne 19; page 12, lignes 4,5 *<br>--- | 1 | **DOMAINES TECHNIQUES RECHERCHES (Int. Cl.4)** |
| A | EP-A-0 157 601 (NATIONAL RESEARCH)<br>* Page 3, lignes 15,16; revendication 3 *<br>----- | 6-8 | A 61 L 27/00<br>A 61 L 31/00<br>B 29 C 55/30 |

Le présent rapport a été établi pour toutes les revendications

| Lieu de la recherche | Date d'achèvement de la recherche | Examinateur |
|---|---|---|
| LA HAYE | 08-12-1988 | PELTRE CHR. |